# EUROPEAN PATENT SPECIFICATION

(11) **EP 3 570 862 B1**
(45) Date of publication and mention of the grant of the patent: **19.08.2020**
(21) Application number: 18705493.7
(22) Date of filing: 17.01.2018
(51) Int. Cl.: A61K 31/7048, A61K 31/7032, A61K 36/752, A61K 36/899, A61K 36/28, A61K 36/605, A61K 36/63, A61K 36/73, A61K 36/80, A61P 27/02, A61P 27/10, A61P 43/00, A61K 9/20, A61K 9/48, A61K 9/16, A23L 5/00, A23L 33/10, A23L 33/105

(54) **COMPOSITIONS BASED ON ACTIVE PRINCIPLES OF PLANTS ORIGIN HAVING EYE PROTECTION ACTION**
ZUSAMMENSETZUNGEN AUF BASIS VON WIRKSTOFFEN PFLANZLICHEN URSPRUNGS MIT AUGENSCHUTZWIRKUNG
COMPOSITIONS À BASE DE PRINCIPES ACTIFS D'ORIGINE VÉGÉTALE AYANT UNE ACTION PROTECTRICE SUR LES YEUX

(30) Priority: 17.01.2017 IT 201700004599
(43) Date of publication of application: 27.11.2019
(73) Proprietor: Safenat S.r.l., 95125 Catania (IT)
(72) Inventor: BONINA, Francesco Paolo, 95125 Catania (IT)
(74) Representative: Calogero, Ida
(86) International application number: PCT/IB2018/050277
(87) International publication number: WO 2018/134738

(56) References cited:
- DATABASE WPI Week 201445 Thomson Scientific, London, GB; AN 2014-M33144 XP002774034, -& JP 2014 118378 A (ORIZA YUKA KK) 30 June 2014 (2014-06-30)
- YONG WANG ET AL: "Cyanidin-3-glucoside and its phenolic acid metabolites attenuate visible light-induced retinal degeneration in vivo via activation of Nrf2/HO-1 pathway and NF-[kappa]B suppression", MOLECULAR NUTRITION & FOOD RESEARCH, vol. 60, no. 7, 6 May 2016 (2016-05-06), pages 1564-1577, XP055408965, DE ISSN: 1613-4125, DOI: 10.1002/mnfr.201501048
- MONICA MOSCA ET AL: "Ocular tissues and fluids oxidative stress in hares fed on verbascoside supplement", INTERNATIONAL JOURNAL OF FOOD SCIENCES AND NUTRITION, vol. 65, no. 2, 1 March 2014 (2014-03-01), pages 235-240, XP055250133, GB ISSN: 0963-7486, DOI: 10.3109/09637486.2013.836742 cited in the application
- AUDREY COUGNARD-GRÉGOIRE ET AL: "Olive Oil Consumption and Age-Related Macular Degeneration: The Alienor Study", PLOS ONE, vol. 11, no. 7, E0160240, 28 July 2016 (2016-07-28), pages 1-17, XP055408963, DOI: 10.1371/journal.pone.0160240
- VINHA A F ET AL: "Phenolic profiles of Portuguese olive fruits (Olea europaea L.): Influences of cultivar and geographical origin", FOOD CHEMISTRY, vol. 89, no. 4, 1 March 2005 (2005-03-01), pages 561-568, XP027770158, ELSEVIER LTD, NL ISSN: 0308-8146 [retrieved on 2005-03-01]

## Description

The present invention relates to nutraceutical or pharmaceutical compositions based on a combination of cyanidin-3-O-glucoside and verbascoside, or extracts of plant origin enriched in the two active ingredients, with a protective action on eyesight.

In recent years, polyphenolic compounds of plant origin have been of particular interest due to their capacity of acting as protectors with respect to sight disorders due to aging and in the treatment and/or prevention of visual pathologies. Among these, numerous studies relate to anthocyanoside compounds, pigmented substances present in various plants in nature ((bilberries, mulberries, blackberries, oranges, black potatoes, black corn, etc.). Between the '60s and '80s, various scientific studies were conducted on the beneficial effects produced by the intake of food rich in anthocyanins on the wellbeing of sight (Kalt W. et al., 2010).

The results obtained so far, even if sometimes controversial, have shown how these substances can act on some molecular biochemical mechanisms involved in vision and in different visual degenerations.

In the *in vitro* study carried out by Matsumoto H. et al. (Matsumoto H. et al., 2003), anthocyanoside compounds, having different chemical structures, were evaluated in the regeneration of rhodopsin, a photosensitive protein present in the rods which, on undergoing a conformational variation from the 11-cis-retinal form to the 11-trans-retinal form, activates a cascade of biochemical events and mediators responsible for visual nerve impulses. From the results obtained by Matsumoto H. et al., it has been observed that among the various anthocyanoside compounds, glucosylated forms, such as cyanidin-3-O-glucoside, induce a greater increase in rhodopsin regeneration kinetics, resulting in improved visual ability and adaptation in the dark (Nakaishi H. et al., 2000). Furthermore, numerous *in vitro* studies conducted using cell models have demonstrated the strong protective effect of anthocyanoside compounds, obtained from various food sources, from damage induced by oxidative stress on retinal pigment epithelial cells (Wang Y. et al., 2015; Liu Y. et al., 2012; Sun M. et al., 2015).

JP2014118378 discloses the use of cyanidin-3-O-glucoside for the treatment of retinal endothelial cell injury.

YONG WANG ET AL: "Cyanidin-3-glucoside and its phenolic acid metabolites attenuate visible light-induced retinal degeneration in vivo via activation of Nrf2/HO-1 pathway and NFkB suppression", MOLECULAR NUTRITION & FOOD RESEARCH, vol. 60, no. 7, 6 May 2016, pages 1564-1577, discloses that cyanidin-3-O-glucoside attenuates visible light-induced retinal degeneration.

It is known, in fact, that the retinal pigment epithelium (RPE) exerts functions essential for the retinal activity and is particularly sensitive to the damage induced by the radical species of oxygen produced in ocular tissues, with consequences that can cause various eye diseases such as senile degenerative maculopathies, retinal detachment or retinal dystrophies.

The effect produced by anthocyanoside compounds and, in particular, cyanidin-3-O-glucoside on the prevention and treatment of ocular degenerations, also seems to be correlated to the protective action exerted by these compounds on another important ocular tissue, retinal ganglion cells.

The *in vitro* and *in vivo* study carried out by Tanaka et al. (Tanaka J. et al., 2013) showed that treatment with an anthocyanoside extract from black rice containing cyanidin-3-O-glucoside, is able to inhibit the overproduction of cellular radical species induced by tunicamycin treatment, and increase cell viability by inhibition of the caspase-3 activation process, with a consequent reduction in potential cell death due to various ocular pathologies such as glaucoma, diabetic retinopathy, retinal ischemia (Kalt W. et al., 2010). There are studies, however, that report conflicting data in literature. The effect of anthocyanosides on vision, in fact, seems to be dose dependent.

According to Levy Y. & Glovinsky Y. in a study on 16 healthy volunteers, the administration of tablets at a dosage of 12, 24 and 36 mg of anthocyanosides extracted from blueberry berries did not show any effects on improving the central vision of the mesopic or scotopic visual field. Analogously, in a study on 18 healthy volunteers, Zadok D. et al. confirmed that the administration of 12 mg and 24 mg of anthocyanosides of blueberry did not result in visual improvements. The lack of effect was also confirmed by Muth E. R. et al. in a double-blind study on contrast sensitivity and night vision after administration of anthocyanosides (160 mg of blueberry extract titrated at 25%).

Verbascoside is a polyphenolic compound, belonging to the family of phenipropanoids, present in various plant species, such as mullein and olive trees, with known anti-inflammatory, antioxidant, antimicrobial, antiviral activities, etc.

Recently, only a few *in vivo* and clinical studies have evaluated the potential protective actions of this bioactive compound on vision. The results obtained have shown that this compound is capable, after oral administration, to protect the tissues and ocular fluids from natural oxidative processes and, moreover, topically to promote the cicatrization of lesions induced on guinea pig corneal epithelia (Mosca M. et al., 2014; Ambrosone L. et al., 2014).

The authors of the present invention have now identified a surprising synergistic action between cyanidin-3-O-glucoside and verbascoside in terms of protective action and increased cell viability of retinal epithelial cells.

An object of the present invention relates to nutraceutical or pharmaceutical compositions comprising cyanidin-3-O-glucoside and verbascoside or plant extracts, each enriched in cyanidin-3-O-glucoside and verbascoside as active ingredients together with physiologically acceptable adjuvants and/or excipients, for use in the prevention and/or treatment of sight disorders or pathologies affecting the retina.

The two active ingredients or their enriched plant extracts present in the compositions or in the combinations according to the invention, are in a mutual weight ratio verbascoside:cyanidin 3-O-glucoside ranging from 1:1 to 1:20, preferably in a mutual weight ratio ranging from 1:4 to 1:10.

A further object of the present invention relates to a combination of cyanidin-3-O-glucoside and verbascoside or plant extracts enriched in cyanidin-3-O-glucoside and verbascoside for simultaneous, sequential or separate use in the prevention and treatment of sight disorders or diseases affecting the retina.

The invention relates in particular to a combination of cyanidin-3-O-glucoside and verbascoside, wherein the two active ingredients or two enriched plant extracts are administered in combination maintaining a mutual weight ratio ranging from 1:1 to 1:20, preferably in a mutual weight ratio ranging from 1:4 to 1:10.

The plant extracts enriched in cyanidin-3-O-glucoside are preferably selected from extracts of black rice (Oryza sativa L.), red orange (Citrus Sinensis L. Osbeck), black mulberry (Morus nigra L.), blackberry (Rubus ulmifolius S.) and black corn (Zea Mays L.) or another plant source containing anthocyanoside substances.

According to the present invention, hybrid varieties of black rice can also be used. According to the present invention, a plant extract enriched in cyanidin-3-O-glucoside refers to a extract having a content of cyanidin-3-O-glucoside ranging from 2-30% parts by weight (w/w).

The plant extracts enriched in verbascoside are preferably selected from extracts of mullein (Verbascum L.), Olive tree (Olea europaea L.) or another plant source containing verbascoside. According to the present invention, a plant extract enriched in verbascoside refers to an extract having a verbascoside content ranging from 0.2-20% parts by weight (w/w). The enrichment of plant extracts in the respective active ingredients is preferably effected by means of nano-ultrafiftration techniques or with the use of adsorbing resins.

The plant extracts enriched in verbascoside or cyanidin-3-O-glucoside according to the invention can be in liquid or powder form (i.e. dried or freeze-dried).

Said sight disorders affecting the retina can be selected from the group consisting of alterations in night vision, glare, retinal photosensitivity, whereas said visual pathologies affecting the retina can be selected from the group consisting of maculopathies, retinopathies, retinal degenerations due to aging and dystrophies. Said visual pathologies are preferably retinitis pigmentosa, diabetic retinopathy or senile degenerative maculopathy. The combinations or compositions according to the invention can obviously be used as adjuvants for the treatment of said visual pathologies in association with *ad hoc* drug therapies.

Said compositions or combinations are preferably suitable for oral administration, even more preferably in the form of oral solutions, oral emulsions, oral suspensions, capsules, tablets or powder. For oral administration, the preferred total concentration range of the two extracts can vary from 10% to 90% by weight of the final composition.

The active ingredients present in the compositions or combinations according to the invention can be administered orally within the following daily dosage ranges in one or more administrations:
cyanidin-3-O-glucoside: from 30 mg/day to 500 mg/day, preferably 100 mg/day verbascoside: from 10 mg/day to 500 mg/day, preferably 25 mg/day.

The present invention is now described for illustrative purposes according to some preferred embodiments with particular reference to the attached figure, in which:

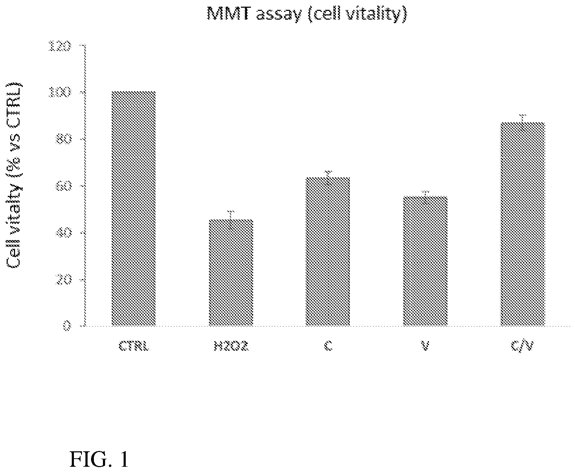

- Figure 1 shows the histogram relating to the percentages of cell viability (MTT assay) of retinal pigment epithelial cells after treatment with H₂O₂ and in the presence of cyanidin-3-O-glucoside extract, verbascoside extract, alone and combined with each other.

By way of example, but not limitative of the present invention, some examples of the compositions according to the invention and their uses are provided hereunder.

### EXAMPLE 1: Preparation of extracts enriched in cyanidin-3-O-glucoside and verbascoside

An exemplary extractive process is described hereunder for obtaining an anthocyanoside plant extract enriched in cyanidin-3-O-glucoside:
Step a: (extraction from black rice or black corn): maceration of the whole grain of rice or corn or pigmented pericarp alone obtained by abrasion of the outer surface of the grain with an aqueous or hydroalcoholic solution (10-60%) at acid pH with hydrochloric acid (0.1% HC1), at room temperature. The maceration process can also be effected with the aid of a Soxhlet extraction system.
Step b: (extraction from black rice or black corn): second extraction on the same vegetable matrix with a new extraction solvent, to favour the recovery of the active substances present.
Step c: recovery and concentration under vacuum of the extracts obtained at a temperature not higher than 50-60°C until the complete elimination of the organic solvent.
Step d: (purification of anthocyanoside substances from red orange juice or blackberry juice or mulberry juice or extract obtained from step c from black rice or black corn): purification of the anthocyanoside compounds by passing the juice or liquid product obtained from step c on an ultrafiltration system (optimal cut off 10,000 daltons) and subsequent concentration by passage on a nanofiltration system (suggested cut off 200 daltons).
Step e: isolation of the anthocyanoside substances by passage of the product obtained from step d on macroporous adsorbent resin Amberlite XAD-7 or another similar adsorbent resin, by elution with an aqueous or hydroalcoholic solution (50-90%) at acid pH with hydrochloric acid (0.1% HCl).
Step f: subsequent drying by spray-drying or freeze-drying using maltodextrin or another technological support.

The anthocyanoside extract obtained has a content of cyanidin-3-O-glucoside, its main constituent, ranging from 2-30% w/w.

An exemplary extraction process is described hereunder for obtaining a polyphenolic plant extract enriched in verbascoside:
Step a: (extraction from vegetable matrix): maceration of the fresh or dried matrix with an aqueous or hydroalcoholic solution (10-60%), acid with a pH value of around 4-5, at a temperature of 40-60°C. The maceration process can also be effected with the aid of a Soxhlet extraction system.
Step b: second extraction on the same vegetable matrix with a new extraction solvent, to favour the recovery of the active substances present.
Step c: recovery and concentration under vacuum of the extracts obtained at a temperature not higher than 50-60°C until the complete elimination of the organic solvent.
Step d: purification of the flavonoid compounds by passage of the liquid product obtained on macroporous adsorbent resin Amberlite XAD-7 or another similar adsorbent resin, by elution with an aqueous or hydroalcoholic solution (30-70%) at acid pH with hydrochloric acid (0.1% HCl).
Step e: concentration under vacuum of the extracts obtained at a temperature not higher than 50-60°C until the complete elimination of the organic solvent and subsequent drying by spray-drying or freeze-drying, with the use of a suitable technological support, e.g. maltodextrin.

The polyphenolic extract obtained has a consent of verbascoside, its main constituent, ranging from 0.2-20% w/w.

### EXAMPLE 2: Formulation examples

Different formulations for oral use (tablets, capsules, powders) are exemplified hereunder, comprising the combination of the anthocyanoside extract (cyanidin-3-O-glucoside) and polyphenolic extract (verbascoside) with a different mutual weight ratio.

### I) Formulation examples for oral use

### - CAPSULES (ratio between verbascoside:cyanidin-3-O-glucoside extracts 1:4)

| **COMPONENTS** | **% w/w** |
|---|---|
| Polyphenolic extract (verbascoside) | 2.5% |
| Anthocyanoside extract (cyanidin-3-O-glucoside) | 10% |
| Lactose | Up to 100 g |

### Preparation method

Mixing the extracts with lactose, according to the rule of geometric dilutions and then proceeding with distribution in capsules.

| **COMPONENTS** | **% w/w** |
|---|---|
| Polyphenolic extract (verbascoside) | 43.7% |
| Anthocyanoside extract (cyanidin-3-O-glucoside) | 43.7% |
| Vegetable magnesium stearate | 0.9% |
| Silicon dioxide | 1.6% |
| Talc | 0.9% |
| Arabic gum | 0.9% |
| Citric acid | 1.0% |
| Coating agents: | |
| - Modified pregelatinized starch | 3.5% |
| - Talc | 2.2% |
| - Lacquer rubber | 0.9% |
| - Glycerol | 0.7% |

### Preparation method

Mixing the anthocyanoside and polyphenolic extracts and proceeding, according to the art, with fluid-bed granulation, using a granulating solution acidified with citric acid and Arabic gum. Mixing the granulate with anti-caking agents (talc, silicon dioxide, magnesium stearate); subjecting the mixture to compression with the use of a tablet press. Applying a first coating of the tablets with lacquer rubber and talc and a subsequent coating with modified pregelatinized starch, glycerol and talc.

### - POWDER FOR ORAL USE (ratio between verbascoside:cyanidin-3-O-glucoside extracts 1:20)

| **COMPONENTS** | **% w/w** |
|---|---|
| Polyphenolic extract (verbascoside) | 1 % |
| Anthocyanoside extract (cyanidin-3-O-glucoside) | 20% |
| Silicon dioxide | 0.67% |
| Sucralose | 0.17% |
| Maltodextrin | Up to 100 g |

### Preparation method

Mixing the extracts with maltodextrin, according to the rule of geometric dilutions and then proceeding with distribution in sachets or bags.

### EXAMPLE 3: Study on the protective activity of anthocyanoside extracts (cyanidin-3-0-glucoside) and polyphenolic extracts (verbascoside) using an in vitro cell model

The objective of the study was the *in vitro* evaluation of the protective action exerted by anthocyanoside plant extracts (cyanidin-3-O-glucoside) and polyphenolic extracts (verbascoside) individually and combined with each other on retinal pigment epithelial cells.

The experimental protocol provided for the use of an *in vitro* cell model capable of evaluating the protective action exerted by the extracts on retinal epithelial cells subjected to a state of oxidative stress.

### MATERIALS AND METHODS

The retinal pigment epithelium (RPE) consists of a layer of pigmented cells, firmly attached to the underlying choroid and to the overlying visual retinal cells, with various functions essential for maintaining the physiological anatomical-functional conditions of the retina. Various visual pathologies, such as retinitis pigmentosa, retinal degenerations due to the physiological aging process, maculopathies and dystrophies, derive and can originate from an altered function of the retinal pigment epithelium.

The ARPE-19 cell line (ATCC CRL-2302) is a line of human retinal pigment epithelium (RPE) cells used as an *in vitro* study model.

These cells were cultured in a 5% CO₂ atmosphere at 37°C, in Dulbecco's Modified Eagle's Medium (DMEM)-HAM F12, with the addition of 10% (v/v) of Fetal Bovine Serum (FBS) and 1% (v/v) of penicillin/streptomycin antibiotics.

### Cell Viability Assay

The RPE cells were trypsinized, counted in a hemocytometer, plated on wells for the MTT assays and, after 24 hours of incubation in a 5% CO₂ atmosphere at 37°C, they were treated for 1 hour with each extract used individually or in a combination, according to what is indicated in Table 1 hereunder. **0.3 mM of H₂O₂** were subsequently added for **24** hours at 37°C.

**Table 1**

| **Sample** | **Composition** | **Concentration** |
|---|---|---|
| C | Cyanidin-3-O-glucoside extract | 100 µg/ml |
| V | Verbascoside extract | 25 µg/ml |
| A/V | Cyanidin-3 -O-glucoside /verbascoside extract | 125 µg/ml |

| | | |
|---|---|---|
| A: Cyanidin-3-O-glucoside extract; V: verbascoside extract; C/V: combination of the two extracts | | |

The cell viability was measured, after treatment with H₂O₂, by the colorimetric assay of tetrazolium salts, which evaluates the capacity of the cells of reducing, by means of mitochondrial succinate dehydrogenase, the bromide of 3-(4,5-dimethylthiazol-2-yl)-2,5-diphenyl tetrazolium (MTT). The MTT enters the cells and passes into the mitochondria where it is reduced to a colored and insoluble product, formazan. To make the color visible, the coloured formazan granules are solubilized by the addition of DMSO. The MTT-cleavage reaction requires the complete integrity of the cell and is proportional to the degree of metabolic activity of the same. The assay provides that after incubation of the cells for three hours, in 5% CO₂ at 37°C, with 20 µl of the solution of the tetrazolium salt, solubilized in medium (5 mg/ml), and 180 µl of medium, the supernatant be removed and 100 µl of DMSO added. The optical density (O.D.) was subsequently measured at a wavelength of 570 nm with a microplate spectrophotometer (Titertek Multiscan, Flow Laboratories). The cell viability was expressed as percentage of optical density with respect to the non-treated control. The experiment was carried out in triplicate for each sample.

### RESULTS

The data obtained from the MTT assays showed that both the anthocyanoside extract (cyanidin-3-O-glucoside) (C) and the polyphenol extract (verbascoside) (V) are able to protect, when used individually, the viability of human retinal pigment epithelial (RPE) cells from the damage induced by treatment with H₂O₂ (Table 1).

The treatment of RPE cells with both extracts used in combination (C/V), causes a significant increase in the cell viability, with a synergic-type effect (see Figure 1 and Table 1). Table 2 hereunder shows the average cell proliferation values (MTT) expressed as cell viability percentage with respect to the control (CTRL) and cell viability percentage (RPE) vs treatment with H₂O₂.

**Table 2**

| **RPE cells** | **cell viability % vs control (viability % vs H₂O₂)** |
|---|---|
| Treatment with H₂O₂ | 45.47 ± 3.74 |
| Cyanidin-3-O-glucoside extract (C) | 63.47 ± 2.91 |
| | (18 ± 1.35) |
| Verbascoside extract (V) | 55.03 ± 2.70 |
| | (9.6 ± 4.03) |
| Cyanidin-3-O-glucoside /verbascoside extract (C/V) | 87.13 ± 3.18* |
| | (41.7 ± 6.7)* |

| | |
|---|---|
| *p<0.05 vs C and V | |

### EXAMPLE 4: Clinical study on healthy volunteers

In the present double-blind randomized study, the in vivo results are illustrated, obtained after oral administration of a preparation containing purified cyanidin 100 mg and verbascoside 25 mg to verify the improvement in sight, the retinal sensitivity and contrast vision.

### PATIENTS

50 healthy volunteers were enrolled, emmetropes (spherical equivalent 0.50 dioptres) between the ages of 30 and 40, 20 men and 20 women (average age 36 years) selected after ascertaining the absence of eye diseases with the following tests:
- Slit lamp examination of the anterior segment and of the fundus
- Applanation tonometry
- Refraction in miosis and cycloplegia
- Natural visual acuity at 6 meters with Sbisà computerized optotype with a brightness of 400 lux
- Contrast visual acuity at 6 meters with Pelli-Robson charts Sbisà computerized optotype with a brightness of 400 lux.

The patients were divided into two groups: 20 men and 20 women. The 20 women patients were respectively divided into two randomized groups, group A and group B.

The 20 patients of group A were used as control and were therefore treated with a capsule preparation containing lactose (inert substance) for 4 weeks.

The 20 patients of group B were treated for 4 weeks with a capsule preparation containing cyanidin-3-O-glucoside (100 mg) and verbascoside (25 mg).

### MATERIALS AND METHODS

The two groups of patients were subjected to three groups of tests.
1) visual tests. Natural and contrast visual acuity before and after treatment to assess possible changes in visual acuity.
2) test for resistance to visual stress. Measurement of glare time and recovery time after glare to evaluate the retinal capacity of adaptation to visual stress.
3) retinal sensitivity test. Examination of the central visual field with white and blue-yellow stimulus.

### Test for resistance to visual stress

In order to evaluate their visual capacity after light stress, the patients were subjected to the glare test and recovery test after glare provided by the Ministry of Health for the issue of driving licenses.

The following tests were performed 4 weeks before and after the administration of the composition according to the invention:
- glare test with a standardized Sbisà optotype
- recovery test after glare with a standardized Sbisà optotype.

The visual acuity tests were performed with standardized computerized Sbisà optotype at a distance of 6 meters with a Snellen Chart with brightness of 400 lux at 50 cm from the screen.

### Glare test with standardized Sbisà optotype

Two lamps placed at the sides of the optotype with 900 lumen light emission (obtained with two 20 watt lamps), to allow additional illumination to that of the 400 - 500 Lux optotype measured at 50 centimeters.

After switching on the two lamps, the subject is asked, in binocular vision, to recognize at least 50% of the letters present from 1/10 onwards.

### Recovery test after glare with standardized Sbisà optotype

The following were used for performing the test: Sbisà computerized optometry with illumination of 300 lux at 50 centimeters. Light source producing an illuminance of 200 lux at 20 cm when in an orthogonal position with respect to the luxmeter sensor (i.e. appropriate light pen).

The test is conducted monocularly on the two eyes with the possible correction of the ametropia and the best result is taken into consideration for the assessment. After adaptation to the dark for a maximum time of one minute, one eye is covered and the light source is placed at 3 cm from the contralateral eye for ten seconds, inviting the subject to stare at it. Once the high beam source has been removed, the lights are turned on again and the subject is asked to instantly read the line corresponding to 4/10, in a maximum time of 30 seconds, the line is considered read if 50% of the letters are recognized in the time established. If the relative line is not read in the maximum time provided, the procedure is repeated in the same way on the other eye.

The eye that has achieved the best result in a time not exceeding 30 seconds, is considered (specific licenses group 2, Ministry of Health). Table B indicates the time necessary for reading the line corresponding to 4/10 measured with a stopwatch and expressed in seconds from the beginning of the test.

### Retinal sensitivity test

In order to assess possible changes in the retinal sensitivity before and after 4 weeks of treatment, the following visual field tests were also performed (Octopus 311 TOP G32 strategy, 30 degrees central).
- white light field of vision
- blue-yellow field of vision

In both types of field of vision, only the **MS** (mean sensitivity) was analyzed, which is the arithmetic mean of the differences between the sensitivity values measured in the various points of the visual field and the normal values corrected for age.

It was decided to perform both visual fields in order to evaluate the different responses of the photoreceptors to the white and blue - yellow stimulus. The use of the blue-yellow perimetry derives from the fact that numerous studies have shown that the S cones, specific for blue, are those affected earlier in glaucoma. The white light, in fact, saturates the responses of all the cones: the defect in response of a single cone system can be compensated by that of the others. The blue-yellow field of vision, viceversa, offers an isolation of 15 dB, the blue yellow ganglion system must therefore lose 15 dB of sensitivity before other cell types intervene in response to the blue stimulus.

Using this method it is possible to evaluate a possible increase in the activity of retinal ganglion cells to support and amplify the luminous message of the photoreceptors.

### Method for examining the visual field

The examination was carried out with Octopus 311 before administration of the composition according to the invention and after 4 weeks of treatment. The examination was effected after a 5-minute adaptation in the dark. In order to avoid false positives, the initial white light test was repeated twice and only the retinal sensitivity indexes of the second field of vision were considered. Subsequently, after a rest period of 30 minutes in normal light and a new adaptation in the dark of 5 minutes, a blue-yellow field of vision was performed which is universally used to test the functionality of retinal ganglion cells. Also in this case, the examination was carried out twice at 10-minute intervals and the retinal sensitivity indices obtained with the second examination were used.

At the end of the 4 weeks of treatment, all patients (group A and group B) were reexamined using the slit lamp of the anterior segment and fundus to ascertain the onset of possible pathologies or side-effects.

### RESULTS

The results obtained are summarized in the following tables.

The patients of group A to whom placebo was administered were compared with the patients of group B treated with the composition according to the invention for 4 weeks.

The results were assessed at the end of the month of administration. During the weeks of administration, no general or ocular side-effects were observed, either in the placebo group or in the group B treated with the composition according to the invention. All the patients completed the month of treatment.

Table 3 summarizes the changes in the natural and contrast visual acuity, before and after the treatment.

Table 4 summarizes the visual stress resistance data measured with the glare test and recovery after glare test before and after treatment.

Table 5 shows the changes in the retinal sensitivity before and after treatment.

In the three tables, the results obtained in group A of patients who took placebo for 4 weeks are always juxtaposed.

### Statistical analysis

The results obtained in the two groups were analyzed statistically with standard deviation and Student's T.

**Table 3**

| Patient | Visual acuity pre Group A | Visual acuity pre Group B | Visual acuity post Group A | Visual acuity post Group B | Contrast pre Group A | Contrast pre Group B | Contrast post Group A | Contrast post Group B |
|---|---|---|---|---|---|---|---|---|
| 1 | 1.0 | 1.0 | 1.0 | 1.0 | 0.8 | 0.8 | 0.8 | 0.9 |
| 2 | 1.0 | 1.0 | 1.0 | 1.0 | 0.8 | 0.8 | 0.8 | 0.9 |
| 3 | 1.0 | 1.0 | 1.0 | 1.0 | 0.9 | 0.8 | 0.9 | 0.9 |
| 4 | 1.0 | 1.0 | 1.0 | 1.0 | 0.8 | 0.8 | 0.9 | 0.9 |
| 5 | 1.0 | 1.0 | 1.0 | 1.0 | 0.8 | 0.9 | 0.8 | 0.9 |
| 6 | 1.0 | 1.0 | 1.0 | 1.0 | 0.8 | 0.9 | 0.8 | 0.9 |
| 7 | 1.0 | 1.0 | 1.0 | 1.0 | 0.8 | 0.9 | 0.8 | 0.9 |
| 8 | 1.0 | 1.0 | 1.0 | 1.0 | 0.8 | 0.8 | 0.8 | 0.8 |
| 9 | 1.0 | 1.0 | 1.0 | 1.0 | 0.8 | 0.8 | 0.9 | 0.8 |
| 10 | 1.0 | 1.0 | 1.0 | 1.0 | 0.9 | 0.8 | 0.8 | 0.9 |
| 11 | 1.0 | 1.0 | 1.0 | 1.0 | 0.8 | 0.8 | 0.9 | 0.9 |
| 12 | 1.0 | 1.0 | 1.0 | 1.0 | 0.8 | 0.8 | 0.9 | 0.9 |
| 13 | 1.0 | 1.0 | 1.0 | 1.0 | 0.8 | 0.8 | 0.8 | 0.9 |
| 14 | 1.0 | 1.0 | 1.0 | 1.0 | 0.8 | 0.8 | 0.9 | 0.8 |
| 15 | 1.0 | 1.0 | 1.0 | 1.0 | 0.9 | 0.8 | 1.0 | 0.8 |
| 16 | 1.0 | 1.0 | 1.0 | 1.0 | 0.9 | 0.8 | 0.9 | 0.9 |
| 17 | 1.0 | 1.0 | 1.0 | 1.0 | 0.9 | 0.8 | 0.9 | 0.9 |
| 18 | 1.0 | 1.0 | 1.0 | 1.0 | 0.8 | 0.8 | 0.8 | 0.9 |
| 19 | 1.0 | 1.0 | 1.0 | 1.0 | 0.9 | 0.8 | 0.9 | 0.9 |
| 20 | 1.0 | 1.0 | 1.0 | 1.0 | 0.9 | 0.8 | 0.9 | 0.9 |
| **Mean** | **1.0** | **1.0** | **1.0** | **1.0** | **0.83** | **0.81** | **0.86** | **0.88*** |
| **± DS** | **0** | **0** | **0** | **0** | **0.05** | **0.04** | **0.06** | **0.04** |

| | | | | | | | | |
|---|---|---|---|---|---|---|---|---|
| *p<0,05 vs pre-treated group Legend: Contr. = contrast | | | | | | | | |

The visual acuity tests substantially showed that the treatment with the composition according to the invention does not improve the central visual acuity beyond ten tenths (1.0), even if with this study it cannot be excluded that the improvement in the visual acuity can occur below ten tenths. Viceversa, the contrast visual acuity improves after 4 weeks of treatment with respect to the pre-treatment measurements.

**Table 4**

| Patient | (1)Glare Group A | (1)Glare Group B | Rec. Group A | Rec. Group B |
|---|---|---|---|---|
| 1 | 0.3 | 0.4 | 29 | 18 |
| 2 | 0.1 | 0.2 | 27 | 22 |
| 3 | 0.2 | 0.3 | 28 | 23 |
| 4 | 0.2 | 0.3 | 30 | 26 |
| 5 | 0.1 | 0.3 | 30 | 28 |
| 6 | 0.1 | 0.3 | 30 | 26 |
| 7 | 0.1 | 0.2 | 25 | 24 |
| 8 | 0.1 | 0.2 | 26 | 25 |
| 9 | 0.1 | 0.2 | 28 | 26 |
| 10 | 0.1 | 0.2 | 28 | 24 |
| 11 | 0.1 | 0.2 | 30 | 24 |
| 12 | 0.1 | 0.2 | 28 | 24 |
| 13 | 0.1 | 0.2 | 30 | 26 |
| 14 | 0.1 | 0.2 | 27 | 24 |
| 15 | 0.1 | 0.2 | 25 | 24 |
| 16 | 0.1 | 0.2 | 27 | 22 |
| 17 | 0.1 | 0.2 | 28 | 23 |
| 18 | 0.1 | 0.2 | 26 | 24 |
| 19 | 0.1 | 0.3 | 30 | 25 |
| 20 | 0.2 | 0.2 | 36 | 27 |
| **Mean** | **0.12** | **0.23*** | **28.40** | **24.25*** |
| **± DS** | **0.06** | **0.06** | **2.46** | **2.15** |

| | | | | |
|---|---|---|---|---|
| *p<0.05 vs placebo (group A) Legend: Glare = glare test group A and group B visual acuity in tenths; Rec. = recovery test after glare group A and group B in tenths necessary for viewing the line of 4/10 (1) Natural visual acuity before glare group A and group B, see table A. | | | | |

The glare and recovery after glare tests show that group B treated with the composition according to the invention has a greater recovery rate of the visual acuity with respect to the control group A.

**Table 5**

| Patient | MS pre b/b Group A | MS pre b/b Group B | MS post b/b Group A | MS post b/b Group B | MS pre b/g Group A | MS pre b/g Group B | MS post b/g Group A | MS post b/g Group B |
|---|---|---|---|---|---|---|---|---|
| 1 | 27.5 | 27.4 | 27.6 | 27.5 | 21.4 | 21.4 | 21.4 | 21.5 |
| 2 | 27.4 | 27.4 | 27.5 | 27.5 | 21.4 | 21.4 | 21.4 | 21.5 |
| 3 | 27.4 | 27.4 | 27.4 | 27.6 | 21.4 | 21.3 | 21.4 | 21.4 |
| 4 | 27.4 | 27.4 | 27.4 | 27.5 | 21.3 | 21.3 | 21.3 | 21.3 |
| 5 | 27.5 | 27.5 | 27.5 | 27.5 | 21.4 | 21.3 | 21.4 | 21.4 |
| 6 | 27.6 | 27.5 | 27.6 | 27.5 | 21.2 | 21.2 | 21.3 | 21.3 |
| 7 | 27.3 | 27.7 | 27.3 | 27.7 | 21.3 | 21.3 | 21.3 | 21.3 |
| 8 | 27.5 | 27.7 | 27.5 | 27.7 | 21.2 | 21.3 | 21.2 | 21.3 |
| 9 | 27.4 | 27.3 | 27.4 | 27.5 | 21.4 | 21.4 | 21.3 | 21.5 |
| 10 | 27.5 | 27.5 | 27.5 | 27.6 | 21.4 | 21.4 | 21.4 | 21.5 |
| 11 | 27.5 | 27.5 | 27.5 | 27.6 | 21.3 | 21.3 | 21.3 | 21.3 |
| 12 | 27.5 | 27.5 | 27.5 | 27.6 | 21.3 | 21.3 | 21.3 | 21.4 |
| 13 | 27.6 | 27.6 | 27.5 | 27.6 | 21.3 | 21.3 | 21.2 | 21.3 |
| 14 | 27.4 | 27.5 | 27.4 | 27.5 | 21.3 | 21.3 | 21.3 | 21.3 |
| 15 | 27.4 | 27.5 | 27.5 | 27.5 | 21.3 | 21.3 | 21.3 | 21.4 |
| 16 | 27.4 | 27.5 | 27.3 | 27.5 | 21.4 | 21.4 | 21.4 | 21.5 |
| 17 | 27.4 | 27.4 | 27.3 | 27.6 | 21.3 | 21.3 | 21.3 | 21.4 |
| 18 | 27.6 | 27.3 | 27.6 | 27.5 | 21.2 | 21.3 | 21.3 | 21.3 |
| 19 | 27.6 | 27.5 | 27.6 | 27.5 | 21.2 | 21.3 | 21.3 | 21.4 |
| 20 | 27.5 | 27.3 | 27.5 | 27.6 | 21.3 | 21.2 | 21.3 | 21.3 |
| **Mean** | **27.47** | **27.47** | **27.47** | **27.55*** | **21.31** | **21.31** | **21.32** | **21.38*** |
| ± **DS** | **0.09** | **0.11** | **0.10** | **0.07** | **0.07** | **0.06** | **0.06** | **0.08** |

| | | | | | | | | |
|---|---|---|---|---|---|---|---|---|
| *p<0.05 vs pre-treated group Legend: MS = mean sensitivity; b/b = visual field with white light stimulus; b/g = visual field with blue-yellow light stimulus. | | | | | | | | |

In both the white and blue-yellow light fields of vision, group B treated with the composition according to the invention showed an improvement in the retinal sensitivity (MS = mean sensitivity) with respect to the control group A.

### Statistics

In conclusion, the visual tests showed an improvement in the contrast vision, starting from a sample of perfectly healthy subjects and with a normal visual acuity of ten tenths. The best perception of contrast visual acuity is a clear indication of improvement in the retinal sensitivity threshold. The subjects treated are able to perceive higher contrast shades than those treated with placebo.

The glare and recovery after glare tests showed that the retinal ability to withstand light stress significantly improves after treatment with cyanidin and verbascoside. This improvement is explained by a higher regeneration rate of rhodopsin after treatment with the compositions according to the invention.

The retinal sensitivity tests showed an improvement in the blue-yellow sensitivity of the rods thanks to a better functioning of the retinal ganglion cells. This datum should be considered together with the improvement in the contrast vision.

In both cases, the effect is mainly due to the increase in the activity of the retinal ganglion cells which amplify the signal of the rods with an increase in the blue-yellow light sensitivity and with an increase in the sensitivity of the cones with an improvement in the contrast vision.

### EXAMPLE 5: Clinical study on patents with eye diseases

Macular oedema is a multifactorial disease due to an alteration in the permeability of macular capillaries. Vascular damage may be due to systemic diseases such as diabetes, renal failure and blood disorders.

In general, all wet macular degenerations are associated with macular oedema especially when damage of the retinal pigment epithelium and Bruch lamina occurs, which anatomically separates the pigmented epithelium and the retinal layers overlying the layer called capillary chorion. This vascular layer in the absence of protection of the bruch lamina spreads the blood inside the retinal layers, dissecting them and separating them from each other until there is a severe reduction in the visual acuity.

In eye diseases, the vascular damage due to diabetes or hypertension causes an increase in the permeabilization of the vessel and consequently the leakage of plasma from the vessel wall.

This anomaly is easily visible in ophthalmology with retinal fluorangiography. With this examination, the retinal blood circulation is photographed after injection of contrast medium (fluorescein).

The damage to the vascular wall continues with a weakening of the capillary structure which, due to the blood pressure, is reduced by creating a microaneurysm.

In diabetic retinopathy, exudates and microaneurysms are always present both in the initial and late phases where other vascular alterations arise.

In this study, the capillary protection activity was evaluated of cyanidin alone and in combination with the anti-inflammatory, anti-oedema activity and inhibition of metalloproteinases of mullein in the following eye diseases:
- Macular oedema
- Diabetic retinopathy
- Dry macular degeneration
- Wet macular degeneration

### MATERIALS AND METHODS

A randomized double-blind study was conducted on 4 groups of 50 patients each, having the same age.

In order to assess the effectiveness of cyanidin and cyanidin-mullein combination, voluntary patients without other eye diseases and general vascular pathologies, were enrolled.

To avoid vascular pharmacological interactions, the 160 patients were not treated with vaso-protector or antiplatelet drugs for at least three months. Diabetic patients were all treated with oral hypoglycemic agents and with fasting blood sugar not exceeding 200 mg/dl for at least three months.

### Groups of patients

The 4 groups of 40 patients each were divided as follows:
Group A: multifactorial macular oedema: average age 58.2 years (15 males and 25 females) affected by chronic macular oedema diagnosed at least 6 months before, due to the following diseases:
   10 patients with post-surgical macular oedema
   20 patients with macular oedema from diabetic retinopathy
   10 patients with macular oedema due to retinal vein occlusion
Group B: 40 patients with non-proliferating diabetic retinopathy: average age 59.9 years (17 males and 23 females)
Group C: 40 patients with dry macular degeneration: a age 65.2 (20 males and 20 females)
Group D: 40 patients with wet macular degeneration: average age 65.4 (18 males and 22 females)

The patients were previously selected for pathology and diagnosis and underwent the following eye exams:
- Slit lamp examination (CSO)
- OCT (OCT-SLO Optos)
- Fluorangiography (Kowa SW50 degrees)
- Natural and corrected visual acuity test (Standardized Computerized Sbisà optotype)

### Protocol

Unbeknown to the examiner and patients, each group of 40 patients was divided into:
20 patients treated with placebo tablets containing only excipients
20 patients treated with cyanidin-mullein tablets (400 mg cyanidin and 100 mg of verbascoside divided into two tablets - 1 tablet containing 200 mg of cyanidin and 50 mg of verbascoside).

One tablet was given for lunch and one for dinner, continuously for three months. Every seven days, the patients were periodically contacted by telephone to ensure the correct implementation of the therapy.

The following clinical parameters were assessed in the 4 groups:
- best correct visual acuity (BSCA) with visual acuity measured in decimals from 0.1 to 1.0
- maximum thickness of the macular retinal oedema (SM) with OCT macular topography
- fundus variations (OCT and flurangiography).

This evaluation was made by comparing OCT and flurangiography before treatment and at the end of treatment.

Possible variations in a positive sense were evaluated with a subjective scale from 1 to 5 (+ sign) and in a negative sense with a scale from 1 to 5 (- sign). Not only was the trend of the visual acuity evaluated, but also the appearance of the retina before and after treatment.

In particular, in the group of patients with diabetic retinopathy, possible reductions in the number and size of retinal exudates and microaneurysms and intraretinal microhemorrhages from capillary fragility, were observed and evaluated, and finally, in fluorangiography, the diffusion of dye from the retinal vessels 3 minutes after the injection of fluorescein and any capillary amputations, indication of retinal ischemia inducing proliferative retinopathy, was evaluated.

### Statistical analysis

The results of the groups treated were compared to the placebo groups, comparing the delta of the BCVA (best correct visual acuity) values and the macular thickness (SM) before and after treatment.

The deltas obtained from the pre- and post-treatment differences of each group (placebo and treated) were statistically analyzed with standard deviation and Student's T.

### RESULTS

The results illustrated in the following Tables 6 and 7 show a clear activity of the cyanidin + verbascoside combination in the improvement of macular oedema in the treated group A compared to the placebo group.

**Table 6**

| | **Placebo** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after 3 months** |
| | | | | | | | |
| 1 | 0.1 | 0.2 | 4 | 385 | 381 | 4 | + |
| 2 | 0.1 | 0.2 | 3 | 397 | 394 | 3 | + |
| 3 | 0.1 | 0.1 | 6 | 405 | 399 | 6 | + |
| 4 | 0.1 | 0.2 | 1 | 403 | 402 | 1 | + |
| 5 | 0.1 | 0.3 | -8 | 389 | 397 | -8 | + |
| 6 | 0.2 | 0.3 | 16 | 383 | 367 | 16 | + |
| 7 | 0.2 | 0.2 | -18 | 370 | 388 | -18 | + |
| 8 | 0.2 | 0.2 | -8 | 449 | 457 | -8 | - |
| 9 | 0.2 | 0.3 | -19 | 447 | 466 | -19 | - |
| 10 | 0.2 | 0.3 | 10 | 456 | 446 | 10 | + |
| 11 | 0.1 | 0.1 | -15 | 400 | 415 | -15 | - |
| 12 | 0.1 | 0.1 | 2 | 380 | 378 | 2 | - |
| 13 | 0.2 | 0.1 | -5 | 454 | 459 | -5 | - |
| 14 | 0.2 | 0.2 | -14 | 356 | 370 | -14 | - |
| 15 | 0.2 | 0.2 | -9 | 421 | 430 | -9 | - |
| 16 | 0.3 | 0.3 | 7 | 349 | 342 | 7 | + |
| 17 | 0.2 | 0.2 | -3 | 299 | 302 | -3 | - |
| 18 | 0.1 | 0.1 | -10 | 340 | 350 | -10 | - |
| 19 | 0.2 | 0.2 | -10 | 356 | 366 | -10 | - |
| 20 | 0.1 | 0.2 | 3 | 321 | 318 | 3 | + |
| **Mean** | **0.16** | **0.20** | **-3.35** | **388.00** | **391.35** | **-3.35** | |
| **DS** | **0.06** | **0.07** | **-1.47** | **43.96** | **45.43** | **-1.47** | |

**Table 7**

| | **Group A** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after 3 months** |
| 1 | 0.1 | 0.6 | 0.5 | 426 | 221 | 205 | +++++ |
| 2 | 0.1 | 0.7 | 0.6 | 455 | 201 | 254 | +++++ |
| 3 | 0.1 | 0.7 | 0.6 | 458 | 199 | 259 | +++++ |
| 4 | 0.1 | 0.7 | 0.6 | 405 | 196 | 209 | +++++ |
| 5 | 0.1 | 0.7 | 0.6 | 406 | 203 | 203 | +++++ |
| 6 | 0.1 | 0.8 | 0.7 | 433 | 186 | 247 | +++++ |
| 7 | 0.2 | 0.8 | 0.6 | 402 | 188 | 214 | +++++ |
| 8 | 0.2 | 0.7 | 0.5 | 344 | 189 | 155 | +++++ |
| 9 | 0.2 | 0.7 | 0.5 | 352 | 192 | 160 | +++++ |
| 10 | 0.2 | 0.8 | 0.6 | 333 | 189 | 144 | +++++ |
| 11 | 0.2 | 0.8 | 0.6 | 321 | 188 | 133 | +++++ |
| 12 | 0.2 | 0.8 | 0.6 | 367 | 178 | 189 | +++++ |
| 13 | 0.2 | 0.8 | 0.6 | 378 | 177 | 201 | +++++ |
| 14 | 0.2 | 0.7 | 0.5 | 375 | 178 | 197 | +++++ |
| 15 | 0.3 | 0.9 | 0.6 | 322 | 180 | 142 | +++++ |
| 16 | 0.3 | 0.9 | 0.6 | 303 | 178 | 125 | +++++ |
| 17 | 0.3 | 0.8 | 0.5 | 389 | 179 | 210 | +++++ |
| 18 | 0.3 | 0.9 | 0.6 | 296 | 188 | 108 | +++++ |
| 19 | 0.3 | 0.9 | 0.6 | 300 | 177 | 123 | +++++ |
| 20 | 0.3 | 0.8 | 0.5 | 344 | 176 | 168 | +++++ |
| **Mean** | **0.20** | **0.78*** | **0.58**** | **370.45** | **188.15*** | **182.30**** | |
| **DS** | **0.08** | **0.09** | **0.01** | **50.62** | **11.52** | **39.10** | |

| | | | | | | | |
|---|---|---|---|---|---|---|---|
| *p < 0.05 vs pre-treatment of the same group **P < 0.05 vs placebo | | | | | | | |

The action of capillary protection and repair of the basement membrane of the capillary, characteristic of cyanidin together with the anti-inflammatory and anti-oedema action of mullein, justifies the reduction of the oedema. The improvement in the visual acuity is also justified by the further action of cyanidin in facilitating the metabolism of the retinal photoreceptors through an acceleration of the rhodopsin cycle. The comparative results between the group treated with the cyanidin-mullein combination and the placebo show a clear effectiveness of the combination of the two substances.

### Data statistical analysis

The statistical analysis of the data obtained in group A shows a significant difference between the BCVA and MS data obtained before and after the treatment (p < 0.05), a condition which is not observed in the placebo. Furthermore, the variation in BCVA and MS expressed as delta BCVA and delta MS in the treated group is significantly different from that obtained in the non-treated group (placebo) (p < 0.05).

The results illustrated in the following Tables 8 and 9 show a clear activity of the cyanidin + verbascoside combination in the improvement of non-proliferating diabetic retinopathy in the treated group B compared to the placebo group.

**Table 8**

| | **Placebo** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after 3 months** |
| | | | | | | | |
| 1 | 0.5 | 0.5 | 0.00 | 203 | 223 | -20 | - |
| 2 | 0.5 | 0.4 | -0.10 | 197 | 242 | -45 | -- |
| 3 | 0.5 | 0.5 | 0.00 | 199 | 221 | -22 | - |
| 4 | 0.4 | 0.4 | 0.00 | 204 | 201 | 3 | - |
| 5 | 0.4 | 0.4 | 0.00 | 200 | 226 | -26 | - |
| 6 | 0.4 | 0.3 | -0.10 | 199 | 233 | -34 | -- |
| 7 | 0.5 | 0.2 | -0.30 | 197 | 222 | -25 | ---- |
| 8 | 0.5 | 0.2 | -0.30 | 198 | 231 | -33 | --- |
| 9 | 0.5 | 0.3 | -0.20 | 201 | 235 | -34 | --- |
| 10 | 0.5 | 0.3 | -0.20 | 203 | 241 | -38 | --- |
| 11 | 0.3 | 0.3 | 0.00 | 230 | 240 | -10 | -- |
| 12 | 0.4 | 0.3 | -0.10 | 241 | 256 | -15 | -- |
| 13 | 0.4 | 0.4 | 0.00 | 221 | 231 | -10 | -- |
| 14 | 0.3 | 0.4 | 0.10 | 189 | 197 | -8 | + |
| 15 | 0.2 | 0.2 | 0.00 | 227 | 231 | -4 | - |
| 16 | 0.3 | 0.3 | 0.00 | 267 | 288 | -21 | - |
| 17 | 0.4 | 0.4 | 0.00 | 236 | 256 | -20 | - |
| 18 | 0.4 | 0.4 | 0.00 | 278 | 276 | 2 | + |
| 19 | 0.5 | 0.5 | 0.00 | 268 | 300 | -32 | - |
| 20 | 0.3 | 0.3 | 0.00 | 296 | 292 | 4 | + |
| **Mean** | **0.41** | **0.35*** | **-0.06** | **222.70** | **242.10*** | **-19.40** | |
| **DS** | **0.09** | **0.09** | **0.00** | **31.90** | **28.27** | **3.62** | |

| | | | | | | | |
|---|---|---|---|---|---|---|---|
| *p<0.05 vs pre-treatment of the same group | | | | | | | |

**Table 9**

| | **Treated** | | | | | | |
|---|---|---|---|---|---|---|---|
| **Pat.** | **Pre BCVA** | **Post BCVA** | **DELTA BCVA** | **Pre SM** | **Post SM** | **DELTA SM** | **Evaluation after 3 months** |
| | | | | | | | |
| 1 | 0.2 | 0.6 | 0.4 | 277 | 221 | 56 | +++++ |
| 2 | 0.3 | 0.7 | 0.4 | 235 | 201 | 34 | +++++ |
| 3 | 0.3 | 0.7 | 0.4 | 245 | 199 | 46 | +++++ |
| 4 | 0.3 | 0.7 | 0.4 | 255 | 196 | 59 | +++++ |
| 5 | 0.3 | 0.7 | 0.4 | 278 | 203 | 75 | +++++ |
| 6 | 0.2 | 0.8 | 0.6 | 223 | 186 | 37 | +++++ |
| 7 | 0.3 | 0.8 | 0.5 | 235 | 188 | 47 | +++++ |
| 8 | 0.3 | 0.7 | 0.4 | 234 | 189 | 45 | +++++ |
| 9 | 0.3 | 0.7 | 0.4 | 257 | 192 | 65 | +++++ |
| 10 | 0.4 | 0.8 | 0.4 | 267 | 189 | 78 | +++++ |
| 11 | 0.4 | 0.8 | 0.4 | 289 | 188 | 101 | +++++ |
| 12 | 0.4 | 0.8 | 0.4 | 267 | 178 | 89 | +++++ |
| 13 | 0.6 | 0.8 | 0.2 | 277 | 177 | 100 | +++++ |
| 14 | 0.6 | 0.7 | 0.1 | 274 | 178 | 96 | +++++ |
| 15 | 0.7 | 0.9 | 0.2 | 210 | 180 | 30 | +++++ |
| 16 | 0.7 | 0.9 | 0.2 | 222 | 178 | 44 | +++++ |
| 17 | 0.4 | 0.8 | 0.4 | 231 | 179 | 52 | +++++ |
| 18 | 0.4 | 0.9 | 0.5 | 221 | 173 | 48 | +++++ |
| 19 | 0.6 | 0.9 | 0.3 | 189 | 170 | 19 | +++++ |
| 20 | 0.5 | 0.8 | 0.3 | 194 | 176 | 18 | +++++ |
| **Mean** | **0.41** | **0.78*** | **0.37**** | **244.00** | **187.05*** | **56.95**** | |
| **DS** | **0.16** | **0.09** | **-0.07** | **29.18** | **12.42** | **16.76** | |

| | | | | | | | |
|---|---|---|---|---|---|---|---|
| *p<0.05 vs pre-treatment of the same group **p<0.05 vs placebo | | | | | | | |

The improvements are perceived both as an increase in the visual acuity and as a reduction in the number of exudates, microhemorrhages and microaneurysms, before and after treatment. The reduction, in fact, of vascular permeability obtained thanks to cyanidin reduces the formation of exudates that are consequent to the diffusion of plasma through the capillary and the synergy with verbascoside also reduces the retinal oedema typical of diabetic retinopathy. In addition to the improvements in the visual acuity, the action of cyanidin and verbascoside causes an overall improvement in the framework of the retinopathy confirmed by the statistical analysis effected on the judgment of the examiner. Also in this group, the sample subjected to placebo did not show any significant improvements, but rather, due to the progression of the retinopathy, there were cases of a reduction in the visual acuity (negative delta values). The statistical data show how, in the treated group, the BVCA and MS values are significantly improved after treatment (p<0.05 post-treatment vs pre-treatment) and this improvement is significantly different from what was observed in the placebo (delta BCVA and delta MV treated vs delta BCVA and delta MS placebo with p<0.05).

The results shown in Tables 10 and 11 indicate a clear activity of the cyanidin+verbascoside combination in the improvement of the dry macular degeneration in the treated group C with respect to the placebo.

This retinal pathology, which currently represents the most frequent cause of hypovision in the Western world, is caused by a series of action mechanisms at present partially unknown.

The improvement in the visual acuity obtained in three months with the combination of cyanidin + verbascoside is probably due to an improvement in the metabolism of the retinal photoreceptors. The powerful capillary-protection and inhibition of retinal proliferation factors (anti-VEGF) demonstrated with verbascoside, however, certainly plays a beneficial role in the long-term treatment of this disabling eye disease.

**Table 10**

| | **Placebo** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after 3 months** |
| | | | | | | | |
| 1 | 0.5 | 0.5 | 0.00 | 203 | 223 | -20 | - |
| 2 | 0.5 | 0.4 | -0.10 | 197 | 220 | -23 | -- |
| 3 | 0.5 | 0.5 | 0.00 | 199 | 203 | -4 | - |
| 4 | 0.4 | 0.4 | 0.00 | 204 | 201 | 3 | - |
| 5 | 0.4 | 0.4 | 0.00 | 200 | 202 | -2 | - |
| 6 | 0.4 | 0.3 | -0.10 | 199 | 198 | 1 | -- |
| 7 | 0.5 | 0.2 | -0.30 | 197 | 204 | -7 | ---- |
| 8 | 0.5 | 0.2 | -0.30 | 198 | 202 | -4 | --- |
| 9 | 0.5 | 0.3 | -0.20 | 201 | 212 | -11 | --- |
| 10 | 0.5 | 0.3 | -0.20 | 203 | 199 | 4 | --- |
| 11 | 0.4 | 0.4 | 0.00 | 188 | 185 | 3 | + |
| 12 | 0.4 | 0.3 | -0.10 | 185 | 185 | 0 | - |
| 13 | 0.5 | 0.4 | -0.10 | 178 | 179 | -1 | - |
| 14 | 0.6 | 0.6 | 0.00 | 189 | 177 | 12 | + |
| 15 | 0.6 | 0.5 | -0.10 | 178 | 179 | -1 | - |
| 16 | 0.5 | 0.5 | 0.00 | 183 | 189 | -6 | - |
| 17 | 0.4 | 0.3 | -0.10 | 178 | 167 | 11 | - |
| 18 | 0.5 | 0.4 | -0.10 | 168 | 166 | 2 | - |
| 19 | 0.7 | 0.6 | -0.10 | 170 | 174 | -4 | - |
| 20 | 0.7 | 0.7 | 0.00 | 178 | 179 | -1 | + |
| Mean | 0.50 | 0.41* | -0.09 | 189.80 | 192.20 | -2.40 | |
| **DS** | **0.09** | **0.13** | **0.04** | **11.74** | **16.61** | **-4.87** | |

| | | | | | | | |
|---|---|---|---|---|---|---|---|
| *p<0.05 vs pre-treatment of the same group | | | | | | | |

**Table 11**

| | **Treated** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after 3 months** |
| | | | | | | | |
| 1 | 0.2 | 0.3 | 0.1 | 232 | 221 | 11 | ++++ |
| 2 | 0.3 | 0.4 | 0.1 | 231 | 201 | 30 | ++++ |
| 3 | 0.3 | 0.4 | 0.1 | 242 | 199 | 43 | ++++ |
| 4 | 0.3 | 0.4 | 0.1 | 236 | 196 | 40 | ++++ |
| 5 | 0.3 | 0.4 | 0.1 | 233 | 203 | 30 | ++++ |
| 6 | 0.2 | 0.4 | 0.2 | 198 | 186 | 12 | +++++ |
| 7 | 0.3 | 0.5 | 0.2 | 199 | 188 | 11 | +++++ |
| 8 | 0.3 | 0.4 | 0.1 | 194 | 189 | 5 | ++++ |
| 9 | 0.3 | 0.4 | 0.1 | 231 | 192 | 39 | ++++ |
| 10 | 0.4 | 0.5 | 0.1 | 237 | 189 | 48 | ++++ |
| 11 | 0.4 | 0.5 | 0.1 | 194 | 188 | 6 | ++++ |
| 12 | 0.4 | 0.5 | 0.1 | 196 | 178 | 18 | ++++ |
| 13 | 0.6 | 0.6 | 0 | 193 | 177 | 16 | + |
| 14 | 0.6 | 0.6 | 0 | 192 | 178 | 14 | + |
| 15 | 0.7 | 0.8 | 0.1 | 199 | 180 | 19 | +++ |
| 16 | 0.7 | 0.8 | 0.1 | 198 | 178 | 20 | +++ |
| 17 | 0.4 | 0.5 | 0.1 | 193 | 179 | 14 | ++++ |
| 18 | 0.4 | 0.6 | 0.2 | 197 | 173 | 24 | +++++ |
| 19 | 0.6 | 0.7 | 0.1 | 196 | 170 | 26 | ++++ |
| 20 | 0.5 | 0.6 | 0.1 | 199 | 176 | 23 | ++++ |
| **Mean** | **0.41** | **0.52*** | **0.11**** | **209.50** | **187.05*** | **22.45**** | |
| **DS** | **0.16** | **0.14** | **-0.02** | **19.12** | **12.42** | **6.70** | |

| | | | | | | | |
|---|---|---|---|---|---|---|---|
| *p<0.05 vs pre-treatment of the same group **p<0.05 vs placebo | | | | | | | |

The treated group shows a significant variation in the BCVA and SM values with respect to the initial values (pre-treatment) and a superior pre-post treatment improvement in a statistically significant manner, with respect to the placebo (delta BCVA and delta MS values). The results indicated in the following Tables 12 and 13 show a clear activity of the cyanidin + verbascoside combination in the improvement of the most disabling form of macular degeneration, the wet form.

The wet form can be found in about 25% of the patients suffering from macular degeneration. The formation of new subretinal vessels, typical of the wet form, causes a total disruption of the delicate macular retinal structure, accelerating the destruction process of the retinal photoreceptors.

**Table 12**

| | **Placebo** | | | | | | |
|---|---|---|---|---|---|---|---|
| **atient** | **Pre BCVA** | **Post BCVA** | **DELTA BCVA** | **Pre SM** | **Post SM** | **DELT A SM** | **Evaluation after 3 months** |
| | | | | | | | |
| 1 | 0.5 | 0.5 | 0.00 | 203 | 200 | 3 | + |
| 2 | 0.5 | 0.4 | -0.10 | 197 | 199 | -2 | - |
| 3 | 0.5 | 0.5 | 0.00 | 199 | 200 | -1 | - |
| 4 | 0.4 | 0.4 | 0.00 | 204 | 201 | 3 | + |
| 5 | 0.4 | 0.4 | 0.00 | 200 | 200 | 0 | + |
| 6 | 0.4 | 0.3 | -0.10 | 199 | 202 | -3 | - |
| 7 | 0.5 | 0.4 | -0.10 | 197 | 203 | -6 | - |
| 8 | 0.5 | 0.4 | -0.10 | 198 | 201 | -3 | - |
| 9 | 0.5 | 0.4 | -0.10 | 201 | 204 | -3 | - |
| 10 | 0.5 | 0.4 | -0.10 | 203 | 205 | -2 | - |
| 11 | 0.3 | 0.2 | -0.10 | 189 | 197 | -8 | - |
| 12 | 0.3 | 0.2 | -0.10 | 199 | 203 | -4 | - |
| 13 | 0.3 | 0.1 | -0.20 | 197 | 234 | -37 | - |
| 14 | 0.3 | 0.3 | 0.00 | 188 | 186 | 2 | - |
| 15 | 0.2 | 0.2 | 0.00 | 234 | 264 | -30 | - |
| 16 | 0.2 | 0.2 | 0.00 | 313 | 326 | -13 | - |
| 17 | 0.3 | 0.2 | -0.10 | 278 | 279 | -1 | - |
| 18 | 0.3 | 0.2 | -0.10 | 306 | 312 | -6 | - |
| 19 | 0.3 | 0.2 | -0.10 | 295 | 299 | -4 | - |
| 20 | 0.3 | 0.3 | 0.00 | 222 | 225 | -3 | - |
| **Mean** | **0.38** | **0.31** | **-0.06** | **221.10** | **227.00** | **-5.90** | |
| **DS** | **0.11** | **0.12** | **0.01** | **41.16** | **43.55** | **-2.39** | |

**Table 13**

| | **Treated** | | | | | | |
|---|---|---|---|---|---|---|---|
| **Patient** | **Pre BCVA** | **Post BCVA** | **DELTA BCVA** | **Pre SM** | **Post SM** | **DELTA SM** | **Evaluation after three months** |
| | | | | | | | |
| 1 | 0.2 | 0.3 | 0.1 | 236 | 222 | 14 | ++ |
| 2 | 0.3 | 0.3 | 0 | 231 | 201 | 30 | + |
| 3 | 0.3 | 0.4 | 0.1 | 242 | 199 | 43 | ++ |
| 4 | 0.3 | 0.4 | 0.1 | 246 | 198 | 48 | +++ |
| 5 | 0.3 | 0.4 | 0.1 | 243 | 200 | 43 | ++++ |
| 6 | 0.2 | 0.4 | 0.2 | 228 | 187 | 41 | +++++ |
| 7 | 0.3 | 0.4 | 0.1 | 399 | 188 | 211 | ++++ |
| 8 | 0.3 | 0.3 | 0 | 394 | 189 | 205 | + |
| 9 | 0.3 | 0.3 | 0 | 243 | 192 | 51 | + |
| 10 | 0.4 | 0.4 | 0 | 277 | 189 | 88 | + |
| 11 | 0.4 | 0.5 | 0.1 | 197 | 188 | 9 | ++ |
| 12 | 0.4 | 0.5 | 0.1 | 198 | 178 | 20 | +++ |
| 13 | 0.3 | 0.5 | 0.2 | 196 | 177 | 19 | +++ |
| 14 | 0.4 | 0.7 | 0.3 | 197 | 178 | 19 | ++++ |
| 15 | 0.3 | 0.3 | 0 | 199 | 180 | 19 | ++ |
| 16 | 0.3 | 0.4 | 0.1 | 198 | 182 | 16 | ++ |
| 17 | 0.2 | 0.3 | 0.1 | 198 | 180 | 18 | ++ |
| 18 | 0.2 | 0.3 | 0.1 | 191 | 178 | 13 | ++ |
| 19 | 0.2 | 0.3 | 0.1 | 295 | 183 | 112 | ++ |
| 20 | 0.2 | 0.3 | 0.1 | 285 | 180 | 105 | ++ |
| **Mean** | **0.29** | **0.39*** | **0.10**** | **244.65** | **188.45*** | **56.20**** | |
| **DS** | **0.07** | **0.10** | **0.03** | **60.82** | **11.19** | **49.63** | |

| | | | | | | | |
|---|---|---|---|---|---|---|---|
| *p<0.05 vs pre-treatment of the same group **p<0.05 vs placebo | | | | | | | |

### Statistical analysis of the data

The statistical analysis of the data shows how no significant improvement is observed in the placebo group before and after the treatment, whereas a significantly different variation between pre- and post-treatment is obtained for the BCVA and SM parameters in the group treated with the combination in question (p<0.05). Also in this case, in fact, the values of delta BCVA and delta SM obtained in the treated group prove to be significantly different from those obtained in the placebo (p<0.05).

### BIBLIOGRAPHIC REFERENCES

- Kalt W.,Hanneken A., Milbury P., Tremblay F. Recent research on polyphenolics in vision and eye health. J. Agric. Food Chem. 2010, 58, 4001-4007.
- Matsumoto H., Nakamura Y., Tachibanaki S., Kawamura S., Hirayama M. Stimulatory Effect of Cyanidin 3-Glycosides on the Regeneration of Rhodopsin. J. Agric. Food Chem. 2003,51,3560-3563.
- Nakaishi H., Matsumoto H., Tomiga S., Hirayama M. Effects of Black Currant Anthocyanoside Intake on Dark Adaptation and VDT Work-induced Transient Refractive Alteration in Healthy Humans. Altern Med Rev 2000, 5(6):553-562.
- Wang Y., Zhang D., Liu Y., Wand D., Liu J., Ji B. The protective effects of berry-derived anthocyanins against visible light-induced damage in human retinal pigment epithelial cells. J Sci Food Agric 2015; 95: 936-944.
- Liu Y., Song X., Zhang D., Zhou F., Wang D., Wei Y., Gao F., Xie L., Jia G., Wu W., Ji B. Blueberry anthocyanins: protection against ageing and light-induced damage in retinal pigment epithelial cells. British Journal of Nutrition (2012), 108, 16-27.
- Sun M.,Lu X., Hao L.,Wu T., Zhao H., Wang C. The influences of purple sweet potato anthocyanin on the growth characteristics of human retinal pigment epithelial cells. Food & Nutrition Research 2015, 59: 27830.
- Tanaka J., Nakanishi T., Shimoda H.,Nakamura S., Tsuruma K., Shimazawa M., Matsuda H., Yoshikawa M., Hara H. Purple rice extract and its constituents suppress endoplasmic reticulum stress-induced retinal damage in vitro and in vivo. Life Sciences 92 (2013) 17-25.
- Levy Y, Glovinsky Y. The effect of anthocyanosides on night vision. Eye 1998; 12: 967-969.
- Zadok D., Levy Y, Glovinsky Y. The effects anthocyanosides in a multiple oral dose on night vision. Eye (Lond). *1999;13(Pt 6):734-6.
- Muth ER, Laurent HM, Jasper P. The Effect of Bilberry Nutritional Supplementation on Night Visual Acuity and Contrast Sensitivity. Altern Med Rev 2000; 5: 164-173.
- Mosca M., Ambrosone L., Semeraro F., Casamassima D., Vizzarri F., Costagliola C. Ocular tissues and fluids oxidative stress in hares fed on verbascoside supplement. Int J Food Sci Nutr. 2014 Mar;65(2):235-40.
- Ambrosone L., Guerra G., Cinelli M., Filippelli M., Mosca M., Vizzarri F., Giorgio D., Costagliola C. Corneal Epithelial Wound Healing Promoted by Verbascoside-Based Liposomal Eyedrops. Hindawi Publishing Corporation BioMed Research International Volume 2014, Article ID 471642.

## Claims

1. Nutraceutical or pharmaceutical compositions comprising cyanidin 3-O-glucoside and verbascoside or plant extracts, each extract enriched in cyanidin 3-O-glucoside and verbascoside as active ingredients together with physiologically acceptable adjuvants and/or excipients, for use in the prevention and/or treatment of sight disorders or eye diseases affecting the retina.

2. A nutraceutical or pharmaceutical composition for use according to claim 1, wherein the verbascoside and cyanidin-3-O-glucoside or plant extracts enriched in verbascoside and cyanidin-3-glucoside are in a mutual weight ratio ranging from 1:1 to 1:20, preferably in a mutual weight ratio ranging from 1:4 to 1:10.

3. The nutraceutical or pharmaceutical composition for use according to any of claims 1-2, in the form of an oral solution, oral emulsion, oral suspension, capsule, tablet, powder or granulates.

4. The nutraceutical or pharmaceutical composition for use according to any of claims 1-3, wherein the total concentration of the extracts ranges from 10%-90% by weight of the final composition.

5. A combination of cyanidin-3-O-glucoside and verbascoside or plant extracts, each extract enriched in cyanidin 3-O-glucoside and verbascoside for simultaneous, sequential or separate use in the prevention and treatment of visual disorders or eye diseases affecting the retina.

6. The combination of cyanidin-3-O-glucoside and verbascoside or plant extracts, each extract enriched in cyanidin 3-O-glucoside and verbascoside for use according to claim 5, wherein the verbascoside and cyanidin-3-O-glucoside or the plant extracts enriched in verbascoside and cyanidin-3-glucoside are in a mutual weight ratio ranging from 1:1 to 1:20, preferably ranging from 1:4 to 1:10.

7. The composition or combination for use according to any of claims 1 to 6, wherein said plant extract enriched in cyanidin-3-O-glucoside is selected from the group consisting of the extract of black rice (Oryza sativa L.), red orange (Citrus Sinensis L. Osbeck), black mulberry (Morus nigra L.), blackberry (Rubus ulmifolius S.) and black corn (Zea Mays L.).

8. The composition or combination for use according to any of claims 1 to 7, wherein said plant extract enriched in cyanidin-3-O-glucoside has a content of cyanidin-3-O-glucoside within the range of 2-30% parts by weight (w/w).

9. The composition or combination for use according to any of claims 1 to 8, wherein said plant extract enriched in verbascoside is selected from the extract of mullein (Verbascum L.) and olive trees (Olea europaea L.).

10. The composition or combination for use according to any of claims 1 to 9, wherein said plant extract enriched in verbascoside has a content of verbascoside within the range of 0.2-20% parts by weight (w/w).

11. The composition or combination for use according to any of the previous claims, wherein said sight disorders are selected from the group consisting of alterations of night vision, glare and retinal photosensitivity.

12. The composition or combination for use according to any of claims 1-10, wherein said eye diseases are selected from the group consisting of maculopathies, retinopathies, retinal degenerations due to aging and dystrophies.

13. The composition or combination for use according to claim 12, wherein said eye diseases are selected from the group consisting of pigmentosa retinitis, diabetic retinopathy and senile degenerative maculopathy.

14. The composition or combination for use according to any of the previous claims, wherein the cyanidin-3-O-glucoside is administered in a daily dosage ranging from 30 mg/day to 500 mg/day, preferably 100 mg/day and the verbascoside is administered in a daily dosage ranging from 10 mg/day to 100 mg/day, preferably 50 mg /day.

## Patentansprüche

1. Nutrazeutische oder pharmazeutische Zusammensetzungen, umfassend Cyanidin-3-O-glucosid und Verbascosid oder Pflanzenextrakte, wobei jeder Extrakt mit Cyanidin-3-O-glucosid und Verbascosid als Wirkstoffe angereichert ist, zusammen mit physiologisch annehmbaren Hilfsstoffen und/oder Trägerstoffen, zur Verwendung bei der Vorbeugung und/oder Behandlung von Sehstörungen oder Augenkrankheiten, die die Netzhaut betreffen.

2. Nutrazeutische oder pharmazeutische Zusammensetzung zur Verwendung gemäß Anspruch 1, wobei das Verbascosid und Cyanidin-3-O-glucosid oder die Pflanzenextrakte, die mit Verbascosid und Cyanidin-3-glucosid angereichert sind, in einem relativen Gewichtsverhältnis im Bereich von 1:1 bis 1:20, vorzugsweise in einem relativen Gewichtsverhältnis im Bereich von 1:4 bis 1:10 stehen.

3. Nutrazeutische oder pharmazeutische Zusammensetzung zur Verwendung gemäß einem der Ansprüche 1-2, in Form einer oralen Lösung, oralen Emulsion, oralen Suspension, Kapsel, Tablette, einem Pulver oder Granulate.

4. Nutrazeutische oder pharmazeutische Zusammensetzung zur Verwendung gemäß einem der Ansprüche 1-3, wobei die Gesamtkonzentration der Extrakte im Bereich von 10 Gew.-% - 90 Gew.-% der Endzusammensetzung liegt.

5. Kombination von Cyanidin-3-O-glucosid und Verbascosid oder Pflanzenextrakten, wobei jeder Extrakt mit Cyanidin-3-O-glucosid und Verbascosid angereichert ist, zur gleichzeitigen, aufeinanderfolgenden oder getrennten Verwendung bei der Vorbeugung und Behandlung von Sehstörungen oder Augenkrankheiten, die die Netzhaut betreffen.

6. Kombination von Cyanidin-3-O-glucosid und Verbascosid oder Pflanzenextrakten, wobei jeder Extrakt mit Cyanidin-3-O-glucosid und Verbascosid angereichert ist, zur Verwendung gemäß Anspruch 5, wobei das Verbascosid und Cyanidin-3-O-glucosid oder die Pflanzenextrakte, die mit Verbascosid und Cyanidin-3-glucosid angereichert sind, in einem relativen Gewichtsverhältnis im Bereich von 1:1 bis 1:20, vorzugsweise im Bereich von 1:4 bis 1:10 stehen.

7. Zusammensetzung oder Kombination zur Verwendung gemäß einem der Ansprüche 1 bis 6, wobei der Pflanzenextrakt, der mit Cyanidin-3-O-glucosid angereichert ist, aus der Gruppe ausgewählt ist, die aus dem Extrakt von schwarzem Reis (Oryza sativa L.), Blutorange (Citrus Sinensis L. Osbeck), schwarzer Maulbeere (Morus nigra L.), Brombeere (Rubus ulmifolius S.) und schwarzem Mais (Zea Mays L.) besteht.

8. Zusammensetzung oder Kombination zur Verwendung gemäß einem der Ansprüche 1 bis 7, wobei der Pflanzenextrakt, der mit Cyanidin-3-O-glucosid angereichert ist, einen Gehalt an Cyanidin-3-O-glucosid im Bereich von 2-30 % Gewichtsteilen (w/w) aufweist.

9. Zusammensetzung oder Kombination zur Verwendung gemäß einem der Ansprüche 1 bis 8, wobei der Pflanzenextrakt, der mit Verbascosid angereichert ist, aus dem Extrakt von Königskerze (Verbascum L.) und Olivenbäumen (Olea europaea L.) ausgewählt ist.

10. Zusammensetzung oder Kombination zur Verwendung gemäß einem der Ansprüche 1 bis 9, wobei der Pflanzenextrakt, der mit Verbascosid angereichert ist, einen Verbascosidgehalt im Bereich von 0,2-20 % Gewichtsteilen (w/w) aufweist.

11. Zusammensetzung oder Kombination zur Verwendung gemäß einem der vorhergehenden Ansprüche, wobei die Sehstörungen aus der Gruppe ausgewählt werden, die aus Veränderungen des Nachtsehens, Blendung und Lichtempfindlichkeit der Netzhaut besteht.

12. Zusammensetzung oder Kombination zur Verwendung gemäß einem der Ansprüche 1-10, wobei die Augenkrankheiten aus der Gruppe ausgewählt werden, die aus Makulopathien, Retinopathien, Netzhautdegenerationen aufgrund des Alterns und Dystrophien besteht.

13. Zusammensetzung oder Kombination zur Verwendung gemäß Anspruch 12, wobei die Augenkrankheiten aus der Gruppe ausgewählt werden, die aus Retinitis pigmentosa, diabetischer Retinopathie und seniler degenerativer Makulopathie besteht.

14. Zusammensetzung oder Kombination zur Verwendung gemäß einem der vorhergehenden Ansprüche, wobei das Cyanidin-3-O-glucosid in einer Tagesdosis im Bereich von 30 mg/Tag bis 500 mg/Tag, vorzugsweise 100 mg/Tag verabreicht wird und das Verbascosid in einer Tagesdosis im Bereich von 10 mg/Tag bis 100 mg/Tag, vorzugsweise 50 mg /Tag verabreicht wird.

## Revendications

1. Des compositions nutraceutiques ou pharmaceutiques comprenant de la cyanidine 3-O-glucoside et du verbascoside ou des extraits de plante, chaque extrait étant enrichi en cyanidine 3-O-glucoside et en verbascoside en tant que principes actifs conjointement avec des excipients et/ou des adjuvants physiologiquement acceptables, pour une utilisation dans la prévention et/ou le traitement de troubles de la vue ou de maladies oculaires affectant la rétine.

2. Une composition nutraceutique ou pharmaceutique pour une utilisation selon la revendication 1, dans laquelle le verbascoside et le cyanidine-3-O-glucoside ou les extraits de plante enrichis en verbascoside et en cyanidine-3-glucoside sont dans un rapport pondéral mutuel allant de 1/1 à 1/20, préférablement dans un rapport pondéral mutuel allant de 1/4 à 1/10.

3. La composition nutraceutique ou pharmaceutique pour une utilisation selon n'importe lesquelles des revendications 1 à 2, sous la forme d'une solution orale, d'une émulsion orale, d'une suspension orale, d'une capsule, d'un comprimé, d'une poudre ou de granulés.

4. La composition nutraceutique ou pharmaceutique pour une utilisation selon n'importe lesquelles des revendications 1 à 3, dans laquelle la concentration totale des extraits va de 10 % à 90 % en poids de la composition finale.

5. Une combinaison de cyanidine-3-O-glucoside et de verbascoside ou d'extraits de plante, chaque extrait étant enrichi en cyanidine 3-O-glucoside et en verbascoside pour une utilisation simultanée, séquentielle ou séparée dans la prévention et le traitement de troubles visuels ou de maladies oculaires affectant la rétine.

6. La combinaison de cyanidine-3-O-glucoside et de verbascoside ou d'extraits de plante, chaque extrait étant enrichi en cyanidine 3-O-glucoside et en verbascoside pour une utilisation selon la revendication 5, dans laquelle le verbascoside et le cyanidine-3-O-glucoside ou les extraits de plante enrichis en verbascoside et en cyanidine-3-glucoside sont dans un rapport pondéral mutuel allant de 1/1 à 1/20, préférablement allant de 1/4 à 1/10.

7. La composition ou combinaison pour une utilisation selon n'importe lesquelles des revendications 1 à 6, dans laquelle ledit extrait de plante enrichi en cyanidine-3-O-glucoside est sélectionné dans le groupe constitué de l'extrait de riz noir (Oryza sativa L.), d'orange sanguine (Citrus Sinensis L. Osbeck), de mûre noire (Morus nigra L.), de mûre (Rubus ulmifolius S.) et de maïs noir (Zea Mays L.).

8. La composition ou combinaison pour une utilisation selon n'importe lesquelles des revendications 1 à 7, dans laquelle ledit extrait de plante enrichi en cyanidine-3-O-glucoside a une teneur en cyanidine-3-O-glucoside allant de 2 à 30 % parties en poids (w/w).

9. La composition ou combinaison pour une utilisation selon n'importe lesquelles des revendications 1 à 8, dans laquelle ledit extrait de plante enrichi en verbascoside est sélectionné parmi l'extrait de molène (Verbascum L.) et d'oliviers (Olea europaea L.).

10. La composition ou combinaison pour une utilisation selon n'importe lesquelles des revendications 1 à 9, dans laquelle ledit extrait de plante enrichi en verbascoside a une teneur en verbascoside allant de 0,2 à 20 % parties en poids (w/w).

11. La composition ou combinaison pour une utilisation selon n'importe lesquelles des revendications précédentes, dans laquelle lesdits troubles de la vue sont sélectionnés dans le groupe constitué des altérations de la vision nocturne, de l'éblouissement et de la photosensibilité rétinienne.

12. La composition ou combinaison pour une utilisation selon n'importe lesquelles des revendications 1 à 10, dans laquelle lesdites maladies oculaires sont sélectionnées dans le groupe constitué des maculopathies, des rétinopathies, des dégénérescences rétiniennes dues au vieillissement et des dystrophies.

13. La composition ou combinaison pour une utilisation selon la revendication 12, dans laquelle lesdites maladies oculaires sont sélectionnées dans le groupe constitué par la rétinite pigmentaire, la rétinopathie diabétique et la maculopathie dégénérative sénile.

14. La composition ou combinaison pour une utilisation selon n'importe lesquelles des revendications précédentes, dans laquelle le cyanidine-3-O-glucoside est administré dans une posologie quotidienne allant de 30 mg/jour à 500 mg/jour, préférablement 100 mg/jour et le verbascoside est administré dans une posologie quotidienne allant de 10 mg/jour à 100 mg/jour, préférablement 50 mg/jour.
